# EUROPEAN PATENT APPLICATION

(11) **EP 0 626 651 A2**
(43) Date of publication of application: **30.11.1994**
(21) Application number: 94830153.6
(22) Date of filing: 31.03.1994
(51) Int. Cl.: G06F 15/42

(54) **Apparatus for the management of personalized diets operating interactively with users**

(30) Priority: 27.04.1993 IT RM930263
(71) Applicant: CSF ELETTRONICA S.r.l., I-03100 Frosinone FR (IT)
(72) Inventor: Giuliani, Nicola Pio Maria, I-03012 Anagni FR (IT)
(74) Representative: Tonon, Gilberto

(57) **Abstract**

Apparatus for management of personalized diets working interactively with users, comprising an information processing unit operatively associated with a unit determining the weight of the user and with a unit measuring the height of the user; said information processing unit being furthermore associated with visualization means and cooperating with program means to provide the user with information and questions, and furthermore associated with means for inserting data, information, replies sent from the user to said processing unit; program means set up to provide the user with information regarding a diet for the reduction, the increase or the maintenance of body weight, obtained on the basis of the information exchanged by the user and central data processing unit and the calculation means resident in said processing unit.

## Description

The present invention relates to an apparatus for management of personalized diets operating interactively with the users.

More specifically, the present invention relates to an apparatus of the type mentioned above, comprising electronic weighing scales, an instrument measuring the height of the user and interactive operation or dialogue means between the apparatus and the user to allow input of data in addition to weight and height to be supplied to a data processor associated to the apparatus in such a way that the former can calculate automatically, on the basis of recorded parameters, personalized diets for the decrease, maintenance or increase of weight for each user.

The apparatus indicated above can, furthermore, with the support of its basic structure, supply other additional services, as will be illustrated in the following description.

Automatic weighing scales capable of supplying other information in addition to the weight of a person are known from the state of the art. Programs for more or less personalized diets, for use on personal computers or the like, are also known. However, no apparatus is known that unites these two functions and is capable of supplying appropriate information to a number of users unconnected, as far as possible, from subjective factors and capable of providing corrections over a period of time in order to allow the user to reach and maintain that which is commonly known as his "ideal weight".

According to the present invention an apparatus of the type mentioned is provided, as generally illustrated in the characterizing section of claim 1.

The present invention will now be described with reference to presently preferred embodiments thereof, given as non limiting examples, and according to the figures of the enclosed drawings, in which:
figure 1 shows a plan view of the apparatus according to the present invention as a whole;
figure 2 shows a general block diagram for the central processing unit in the apparatus and for the peripheral devices related thereto;
figure 3 shows a block diagram for a microcontroller associated to input and output devices;
figure 4 shows a block diagram for a further microcontroller associated to the user's weight and height sensors.

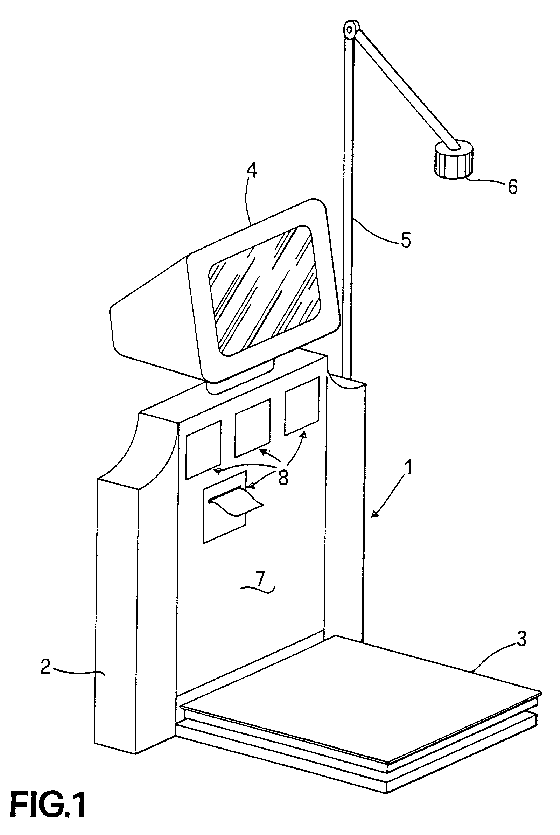

With reference to the drawings, and in particular to figure 1, the apparatus according to the present invention, indicated as a whole with 1, is made up of a base 2 housing the electronics for data control and processing, a platform 3 for weighing the user, a display screen 4, and a column 5 supporting a transducer 6 to measure the height of the user. The base 2 is joined to a panel 7 supporting input/output devices 8, as will be described in greater detail in the following.

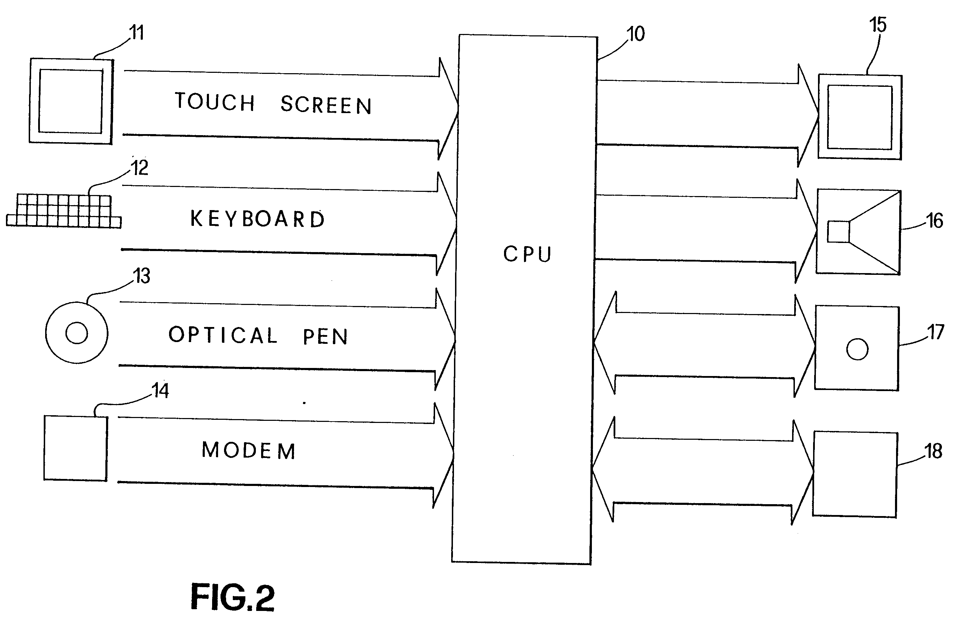

With reference to figures 2, 3 and 4, the electronics of the apparatus shown in figure 1 comprise a central processing unit 10 of the type used for personal computers, joined to data input means comprising a touch-screen 11, a keyboard 12, an optical pen 13 or a modem 14 for optional telephonic connection to a central station (not illustrated) for data loading and optionally for data management. The unit 10 is also connected to a monitor 15, a loudspeaker 16 driven by vocal synthesis means to issue audio messages to the user, a floppy disk drive 17 and a hard disk 18.

These elements will not be described in detail, as their specific structures and methods of operation are well known to any person skilled in information processing technology.

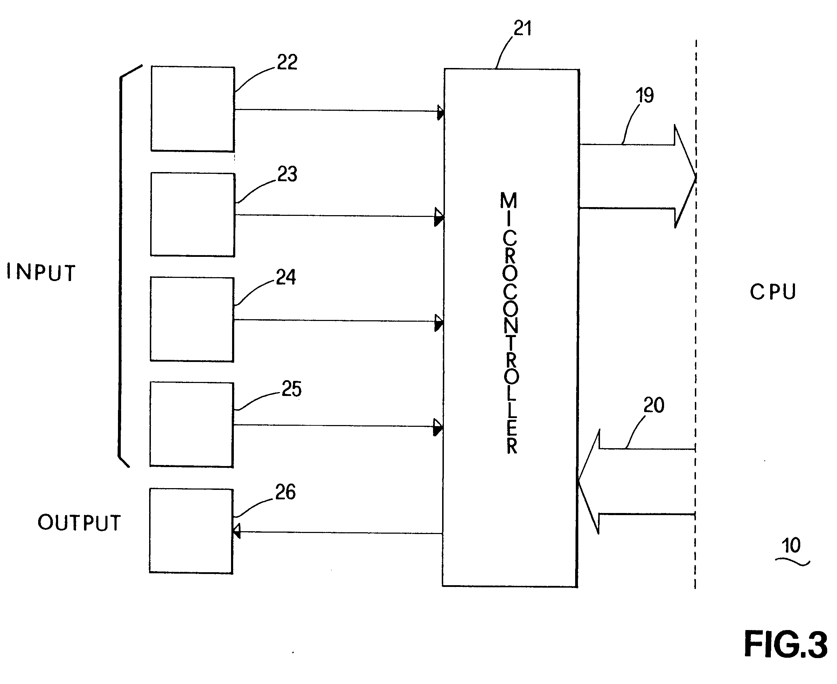

As shown in figure 3, the central processing unit 10 is connected, through the bus 19, 20, to a first microcontroller 21 set up to manage input/output elements such as a first and a second coin slot 22, 23, a banknote controller 25, a badge reader 25 and a printer with relative cutting blade indicated with 26.

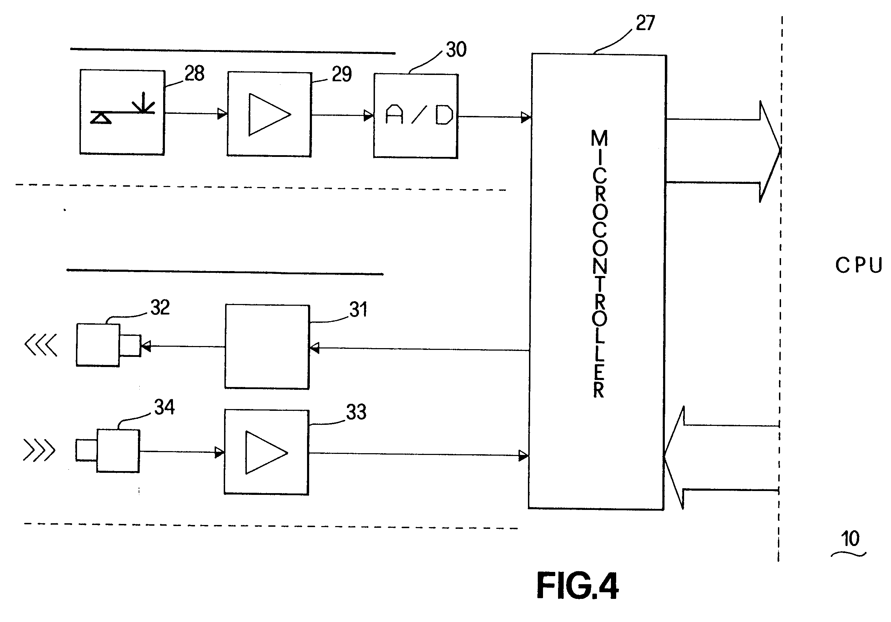

As shown in figure 4, the central data processing unit 10 is furthermore connected to a second microcontroller 27, set up to manage the weighing system comprising a conventional load cell 28, associated with an amplifier-normalizer 29, and with an analog/digital converter 30 having the desired resolution according to the application.

The microcontroller 27 is furthermore associated with a system for measuring the height of the user, comprising in the preferred embodiment a buffer 31 associated with an ultra-sound transmitter 32, and an amplifier 33 associated with a signal receiver 34.

The device for measuring the height of the user is indicated as being of the ultrasound type and preferably of the impulse-echo type, as this kind of apparatus gives more exact results than other distance measuring equipment, such as infra-red ones, which can give incorrect measurements because they "read" the height of the hair and not that of the top of the skull.

The method of operation of the apparatus according to the invention can be divided into two parts: a first part relating to the basic firmware, which is not illustrated, as it depends on the type of the various components used and is in any case within the capabilities of an expert in the field of programmable logic; and a second part relating to the application software, which can have a different form according to the needs and the imagination of the person programming.

In the following an example diagram of an application sequence is given

### EXAMPLE

The apparatus activates an initial Spot when the Height sensor (6) captures a signal in its vicinity, or after every xx minutes of non-activity.
The initial spot is made up as follows:
a screen showing a "logo" appears on the video monitor (4))
Voice:
Personal diet
Pause=)
Your own diet
The screen changes and shows the menu "Choice"
Voice:
calculate your ideal weight
Pause=)
Prepare your own made-to-measure diet
Pause=)
Are you interested?
Pause=)
If so, follow these instructions
At this point, if the user gets onto the weighing platform (3), the data detection cycle is activated.
1) The menu "Choice" remains on screen, but the function keys (8) are activated.
   Voice:
   Do you want to know your ideal weight or do you want to prepare a diet?
   Pause=)
   Reply by touching the screen
2a) If the user chooses to visualize his weight only, the "Weight" screen is displayed
   Voice:
   case a:
   Insert a coin
   case b:
   Insert coins
2b) If the user chooses Weight and Diet, the "Weight and Diet" screen is displayed
   Voice:
   case a:
   Insert the token
   case b:
   Insert notes
3) The screen changes and the "Sex" screen is displayed
   Voice:
   Man or Woman?
   Pause=)
   Reply by touching the screen
4) If the Subject is a woman, the "Particular conditions" screen is displayed
   Voice:
   Reply by touching the keys and then confirm
5) Display the "Bone Structure" screen
   Voice:
   What type of bone structure do you have?
   Pause=)
   Reply by touching the screen
6) If the subject is a woman, display the "Shoe Size" screen
   Voice:
   What size shoes do you wear?
   Pause=)
   Reply by touching the screen
7) Display the "Activity" screen
   Voice:
   What type of physical activity do you do?
   Pause=)
   Reply by touching the screen
8) Display the "Age" screen
   Voice:
   Type your age on the keyboard and then confirm
9) Display the "Measurements" screen
   Gross measurements must be displayed on this screen.
   Voice:
   a) I am reading your height, please hold your head up
   The machine then proceeds to measure height, and at the end of the operation passes on to the next step.
   Voice:
   b) I am reading your weight, please stand on the center of the scales
   ** If, when choosing, the user has chosen to prepare the diet, pass directly on to step 12**
10) If the user has chosen weight only, display the "Data" screen. Warning: there is one screen for overweight subjects and another for underweight ones.
   Voice:
   Do you want me to prepare a made-to-measure diet?
   Pause=)
   Reply by touching the key
10a) If the subject answers NO, exit the program and go directly to step 17a.
10b) If the subject answers YES, proceed with the following step
11) Display the "Weight and Diet" screen (requires the Diet token)
   Voice: (see step 2b)
12) Display the "Physiopathologies" screen
   Voice:
   You can make a maximum of five choices, or none at all
   Pause=)
   Reply by touching the keys and then confirm
13) Display the "Diet" screen (indicates the type of diet)
   Voice:
   What type of diet would you like?
   Pause=)
   Reply by touching the screen
14) Display the "Data" screen.
   N.B.: There are two screens, one showing when the user is overweight and the other showing when the user is underweight.
   Voice:
   Check the information and answer by touching the key
   If the answer is affirmative, proceed with the following step. Otherwise, the user is returned through the cycle in which data regarding Age, Sex, Structure and Activity are requested; for the screens and vocal instructions to be used, see the descriptions given above for the single steps.
15) From this point the "Information/Advice/ Warnings" sequence of screens commences
   Screen A)
      Voice:
      I am preparing your diet
      Pause=)
      Please remember that this diet is strictly personal
      Pause=)
      I suggest you follow it for a maximum of two weeks.
      After that I must prepare you a new one.
   Screen B)
      Voice:
      To give you the greatest possible choice I have grouped together the foods in menus.
      Pause=)
      You must choose, according to your taste, one food in each menu.
   Screen C)
      Voice:
      You can distribute and eat the foods chosen whenever you like during the day.
   Screen D)
      Voice:
      I recommend you never miss out on breakfast
      Pause=)
      There are no particular limits on how much water, salt, spices and medicine prescribed by your doctor you use.
   Screen E)
      Voice:
      Remember that the foods marked with an asterisk are particularly suited for your diet.
      Pause=)
      I advise you to follow your diet under the control of your doctor.
16) Display the "Personal Diet" screen
   Voice:
   Collect the diet and put it into the special folder
17) The program ends by displaying the "Diet" logo once again
   Voice:
   Personal Diet
   Pause=)
   Your own diet
   Pause=)
   A new service from your Chemist.
17a) If the user has chosen Weight alone, the program ends by displaying the "Diet" logo screen
   Voice:
   Please collect your ticket
   Pause=)
   Personal Diet
   Pause=)
   Your own diet
   Pause=)
   A new service from your Chemist.

It must be noted that in the above example no indication has been given of the graphics relating to the various screens, as the contents thereof can be immediately understood from the context, and the actual graphic construction is a matter of choice when preparing the application software, said choice being beyond the scope of protection of the present invention.

Furthermore, in addition to what is indicated in the example given above, it is possible to envisage a number of variants and/or the addition of other functions, both interactive or of a passive advertizing nature, which can be developed on the basis of specific considerations that depart from the scope of the present description. It must be noted, furthermore, that one or more monitors can be provided, operating for at least part of the time in parallel with the monitor of the apparatus, with "video window" type functions of a kind that is known **per se.**

## Claims

1. An apparatus for management of personalized diets operating interactively with users, characterized in that it comprises an information processing unit operatively associated with a unit determining the weight of the user and with a unit measuring the height of the user; said information processing unit being furthermore associated with visualization means and cooperating with program means to provide the user with information and questions, and furthermore associated with means for inserting data, information, replies sent from the user to said processing unit; program means set up to provide the user with information regarding a diet for the reduction, the increase or the maintenance of body weight, obtained on the basis of the information exchanged by the user and central data processing unit and the calculation means resident in said processing unit.

2. An apparatus according to claim 1, characterized in that said information regarding a diet is presented to the user on said screen.

3. An apparatus according to claims 1 or 2, characterized in that it comprises, as well as said screen, printer means to supply the user with diet data on paper.

4. An apparatus according to claims 1 to 3, characterized in that said means for data insertion by the user comprise means of the "touch-screen" type, associated with said screen, and/or a keyboard, and/or an optical pen.

5. An apparatus according to one or more of the preceding claims, characterized in that means are provided for vocal communication between said apparatus and the user.

6. An apparatus according to one or more of the preceding claims, characterized in that said height measuring means are not of the type coming into mechanical contact with the user.

7. An apparatus according to claim 6, characterized in that said height measuring means are of the ultrasound type.

8. An apparatus according to one or more of the preceding claims, characterized in that said data processing unit is associated with means for collecting money.

9. An apparatus according to one or more of the preceding claims, characterized in that said central data processing unit is set up to operate with interactive program means having an architecture of the type illustrated in the example enclosed with the preceding description.

10. An apparatus according to one or more of the preceding claims, characterized in that said program means comprise interactive type functions not connected to the diet problem, and/or of an advertising nature.

11. An apparatus according to one or more of the preceding claims, characterized in that said monitor is associated with auxiliary monitors operating at least partially in parallel with the monitor of the apparatus, with "video window" type functions.

12. An apparatus for the management of personalized diets working interactively with users, according to one or more of the preceding claims and substantially as described and illustrated with reference to the figures of the enclosed drawings.
